# EUROPEAN PATENT SPECIFICATION

(11) **EP 0 436 707 B1**
(45) Date of publication and mention of the grant of the patent: **13.03.1996**
(21) Application number: 90911972.9
(22) Date of filing: 06.07.1990
(51) Int. Cl.: C07D 307/62

(54) **METHOD OF PRODUCING DIPOTASSIUM-ASCORBATE-2-SULPHATE**
VERFAHREN ZUR HERSTELLUNG VON DIKALIUM-ASKORBAT-2-SULPHAT
PROCEDE DE PRODUCTION D'ASCORBATE-2-SULFATE DE DIPOTASSIUM

(43) Date of publication of application: 17.07.1991
(73) Proprietor: PFIZER INC., New York, N.Y. 10017-5703 (US)
(72) Inventor: GUBEROVIC, Zeljko, CH-7000 Chur (CH); HOHNJEC, Marijan, CH-7000 Chur (CH); KUFTINEC, Josip, CH-7000 Chur (CH); OKLOBDZIJA, Milan, CH-7000 Chur (CH)
(74) Representative: Hegner, Mogens
(86) International application number: PCT/DK90/00174
(87) International publication number: WO 91/00859

(56) References cited:
- US-A- 3 954 809
- CHEMICAL ABSTRACTS, Volume 87, No. 10, 5 September 1977, (Columbus, Ohio, US), M. NEZU et al: "Di(alkali metal) L-Ascorbic-2-Sulfate", see page 332, Abstract 73386M, & Japn Kokai 77 25,010 (Kumai Chemical Industry Co. Ltd)

## Description

The present invention relates to a method of producing dipotassium ascorbate-2-sulphate of the formula 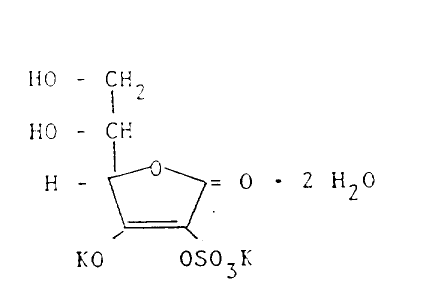 Dipotassium ascorbate-2-sulphate (and the corresponding dihydrate) is a vitamin C derivative which is (somewhat) more stable than vitamin C itself. Among other purposes, the compound is used as an additive in fish food (A.K. Soliman, K. Jauncey, R.J. Roberts; Aquaculture 60;73, (1987). It is a metabolic product of vitamin C which is formed in fish as vitamin C reserve. Vitamin C shortage leads to the deficiency disease scurvy. Fishes such as e.g. salmon possess the enzyme (sulfatase) required to split off the sulphate group, whereby their organism can re-form the pure vitamin from the stored derivative.

It is known from P.A. Sieb et al., J. Chem. Soc. Perkin I, 1120 (1974) how to prepare ascorbic acid being sulphated in position 2 by the reaction of ascorbic acid protected in positions 5 and 6 with pyridine-sulfur trioxide complex in a dimethylformamide solution.

It is known from US Patent Specification No. 3,954,809 how to prepare L-ascorbate-2-sulphate also from unprotected ascorbic acid or its salts by the reaction with a trimethylamine-sulfur trioxide complex in aqueous solution and in the presence of a sufficient amount of a free base, in particular sodium hydroxide, at a pH of 10-10.5. Owing to the presence of a free base, the negative charge of the oxygen atom in position 3 is shifted to the oxygen atom in position 2, as a result of which the latter becomes sulphated in a more reactive and selective way. The thus known method uses a highly diluted solution (ascorbic acid concentration of approximately 80 g/l). In order for the sulfation reaction to proceed to completion, the trimethylamine-sulfur trioxide complex is added in a fairly high excess (over 50%). During the hydrolysis of this excess of sulfur complex, however, there is a parallel formation of not inconsiderable amounts of inorganic sulphates. For reasons of purity these inorganic sulphates cannot be left in the final product. The same applies to the trimethylamine formed during the reaction and being present in solution as well as the unconverted complex still present. In the known method, the separation of the trimethylamine occurs by increasing the pH to approximately 12.5 and subsequent evaporation or by passing the reaction mixture through a column with a cation exchange resin. Separation of the inorganic sulphate, in the form of barium sulphate, is effected by the addition of barium hydroxide. After the addition of methanol and filtration, barium ascorbate-2-sulphate is finally obtained in crystalline form. This barium salt, however, is toxic to fish. With a view to its optional use in fish food, this barium salt would therefore first have to be converted to the far more suitable potassium salt. This is possible in principle through the addition in water of K₂SO₄ to a solution of the barium salt obtained, separation by means of filtration of the barium sulphate being formed, followed by evaporation of the filtrate.

Another possibility of transforming the barium ascorbate-2-sulphate into the corresponding dipotassium salt, described by B.M. Tolbert et al. in Ann. N.Y. Acad. Sci./1975/,258, 48-69, consists in passing an aqueous solution of barium ascorbate-2-sulphate through a column containing DOWEX 50-K⁺.

According to the method described above, it is of course possible to prepare diposassium ascorbate-2-sulphate dihydrate of very high purity. Particularly with regard to the very costly purification of the final product, preparation of this product in a commercial scale for use as additive in fish food (in accordance with the known process) would not, however, be expedient because this method is much too uneconomical.

Furthermore, with the addition of the sulphur reagent in portions, as required by the known method, contamination of the environment with trimethylamine is also difficult to avoid.

An essentially identical method is disclosed in Japanese Kokai 77:25010 (Chemical Abstracts Vol. 87, No. 10, 1977, Abstr. No. 73386m) in which di-(alkali metal)-L-ascorbate-2-sulphates are prepared by reacting L-ascorbic acid and a tertiary amine-SO₃-complex in basic solution (pH 10) with heating to approximately 70°C. As above, use is made of strongly diluted solutions (ascorbic acid concentration about 100 g/l), and the process consequently involves the same disadvantages. It is possible to achieve a degree of purity of 99.6%, but the purification of the product is too uneconomical for the process to be profitable in large scale for producing dipotassium ascorbate-2-sulphate for use as additive in fish food.

It is the object of the present invention to provide a method of producing dipotassium ascorbate-2-sulphate dihydrate sufficiently pure for fish food which can be carried out economically and with high yield on a commercial scale. Furthermore, according to the method contamination of the environment by the trimethylamine-sulphur trioxide complex should be avoidable.

According to the invention this and other objects are achieved by a method characterized by what is state in the characterizing portion of claim 1. Preferred embodiments of the present invention are stated in the dependent claims.

In the method according to the invention, trimethylamine-sulphur trioxide complex is thus added in only slight molar excess to a concentrated solution of potassium L-ascorbate in water, and the pH of this reaction mixture is subsequently adjusted to a value between 9.5 and 10.5 by successive additions of potassium hydroxide and maintained at this value for the duration of the reaction.

By using a concentrated potassium ascorbate solution, a significant advantage is that the trimethylamine, which is readily soluble in water, escapes during the reaction. Thus it does not, as in the known method, remain in solution and therefore does not require costly separation of the trimethylamine. Furthermore, the high concentration results in high production capacity and optimal utilization of the process plant being used.

Owing to the fact that the trimethylamine-sulphur trioxide complex is added to the concentrated solution of potassium L-ascorbate in only slight molar excess, virtually no unconverted complex remains in the reaction mixture at the end of the reaction time. Nor can inorganic sulphates parallelly form in large amounts. As a consequence of the trimethylamine already having escaped, the desired final product can be crystallized out directly by the simple addition of an alkanol, in particular ethanol. The major portion of the inorganic sulphate then remains in solution. Approx. 1-2% of inorganic sulphates can still be detected in the final product, which is not otherwise disturbing in view of the intended use of the final product as an additive in fish food. Thus, no longer separately required with the method pursuant to the invention as compared to the known method described above is the separation of trimethylamine, the separation of the unconverted complex, the separation of the inorganic sulphates and, finally, also the transformation of the toxic barium salt to the desired potassium salt. The latter is owing to use of potassium L-ascorbate in aqueous solution as starting material and to adjustment of the pH in the basic range by means of potassium hydroxide.

Setting the pH at 10 for the duration of the reaction is effected in the present invention for the same reason as in the known method, in order to sulphate the oxygen atom selectively in position 2.

The concentration of the solution of potassium ascorbate in water is preferably set at a level ranging from 500 to 700 g/l.

The molar excess of the trimethylamine-sulphur trioxide complex added to the aqueous potassium ascorbate solution should not be more than 8%, and preferably only 6%.

The temperature of the reaction mixture is preferably set at a value ranging from 57°C to 65°C and is preferably raised linearly during the course of the reaction from the above-mentioned lower temperature to the above-mentioned upper one, the latter in view of the subsequent recovery of the escaping trimethylamine referred to below.

The entire amount of trimethylamine-sulphur trioxide complex can be added to the aqueous potassium ascorbate solution at the start of the reaction.

Instead of starting out with potassium L-ascorbate, the concentrated solution of potassium ascorbate in water can avantageously also be prepared by mixing L-ascorbic acid with water and neutralizing this mixture with potassium hydroxide. To protect the ascorbic acid, the temperature should be maintained below 25°C.

As already mentioned above, the trimethylamine escaping from the reaction mixture during the reaction can be recovered. To that end, it is preferably absorbed in a chlorinated organic solvent, in particular 1,2-dichloroethane, chloroform, carbon tetrachloride, or methylene chloride (dichloromethane). This can be effected in an absorption column connected to the reaction vessel being used in which the absorbent circulates well cooled. The cooling should maintain the temperature of the absorbent below approximately 0°C. Setting the temperature of the reaction mixture (in the reaction vessel) at 57°C to begin with and subsequently increasing it to 65°C is of advantage because this prevents too vigorous a generation of trimethylamine, possibly exceeding the capacity of the absorption column, at the start of the reaction.

By using the above-mentioned absorbent or solvent for collecting the escaping trimethylamine, it is possible to prepare again from the solution obtained the trimethylamine-sulphur trioxide complex required as reagent for the present method. An especially suitable method and one that can be carried out economically even on a commercial scale consists in adding chlorosulfonic acid to the solution containing the trimethylamine and to pass gaseous ammonia through the resulting reaction mixture for the purpose of recovering amine from the amine hydrochloride previously formed as a by-product. The amine can be recovered by means of ammonia because amine hydrochloride is more soluble than ammonium chloride. The amine recovered reacts with chlorosulfonic acid still present to form the desired R₃N:SO₃ complex. The ammonium chloride formed can be dissolved by the addition of water and stirring. Finally, the desired tertiary amine-sulphur trioxide complex can be separated by filtration, washed and, after drying, obtained as dry final product. During the reaction the temperature is preferably set between 0° and 30°C. Ammonia is passed into the said reaction mixture until the pH of the latter is alkaline.

The invention is illustrated in greater detail by way of the below examples.

### EXAMPLE 1

The first portion of a solution of 339 g (6 mols) of potassium hydroxide in 530 ml of water is added to a mixture of 500 g (2,84 mols) of ascorbic acid and 300 ml of water while stirring and cooling at a temperature below 25°C until the pH is 8.5. Then 420 g of trimethylamine-sulphur trioxide complex (3 mols) is added to the potassium ascorbate solution formed. In an absorber system connected with the reaction vessel containing the above solution 2000 ml of dichloromethane cooled to -10°C is circulated. Care is taken that the temperature in the absorber does not rise above approximately 0°C. Now the temperature in the reaction vessel is raised to 57°C. The pH is set at 10 by the addition of another portion of the potassium hydroxide solution. The sulfation reaction then proceeding requires about two hours. During this time, the pH is maintained at the above-mentioned value by addition of the remaining portion of the potassium hydroxide solution and the temperature is slowly increased to 65°C. The reaction is completed when the entire quantity of potassium hydroxide solution prepared has been added.

The reaction mixture in the reaction vessel is now cooled to 15°C. Then 2300 ml of ethanol (96%) is added for a period of 3 hours, while the temperature is simultaneously lowered to 5°C. The suspension forming as a result of the crystallization of the desired final product is still stirred for another hour before being filtered. The filtration residue containing the desired final product in crystalline form is further washed with 1400 ml of ethanol (70%).

In a trial experiment using the procedure described above, 960 g of dried dipotassium ascorbate-2-sulphate could thus be obtained as a white powder with a melting point of 150-152°C, corresponding to a yield of 92%. The purity of the product was established (by HPLC analysis) to be 98.6%.

In the same trial experiment 153 g of absorbed trimethylamine, corresponding to 85% of the theoretical amount, could be detected in the dichloromethane circulating in the absorber system after the reaction.

Following is an example of how trimethylamine-sulphur trioxide complex can be recovered from a solution of trimethylamine in dichloromethane:

33.5 ml (0.507 mols) of chlorosulfonic acid is slowly added to an ice-cooled and well-stirred solution of 28.5 g (0.5 mols) of trimethylamine in 150 ml of dichloromethane. The temperature is maintained at a level below 20°C. The first half of the chlorosulfonic acid reacts highly exothermically and should therefore be added very slowly, while the second half requires less attention. After the entire quantity of chlorosulfonic acid has been added, gaseous ammonia is passed through the resulting solution until the pH becomes basic. While stirring vigorously, 80 ml of water is added to the suspension formed. After 15 minutes, the solid product is filtered off, washed three times with 25 ml of water each time, and finally dried.

In accordance with the procedure described, it should be possible to prepare 55.5-66.5 g, equivalent to a yield of 80-94%, of dry final product (trimethylamine-sulphur trioxide complex).

### EXAMPLE 2

1500 ml of the reaction mixture in accordance with Example 1 is cooled to 0°C after the end of the reaction (likewise carried out in accordance with Example 1). Seed crystals of pure dipotassium ascorbate-2-sulphate are added and the mixture is stirred for three hours. The crystals that have formed during this time are separated by filtration, washed with 500 ml of ethanol (70%), and dried at 60°C under reduced pressure.

It was possible in a trial experiment to obtain 640 g (61%) of dry final product in this manner with a purity of 99.83% (HPLC analysis).

The mother liquor is then concentrated to 400 ml and the temperature reduced to 5°C. Under constant stirring, 400 ml of ethanol (96%) is added successively over a period of 2 hours.

Another 310 g (30%) of the desired final product could thus be obtained in the above trial experiment with a purity of 97.8% (HPLC analysis).

## Claims

1. Method of producing dipotassium ascorbate-2-sulphate dihydrate of the formula 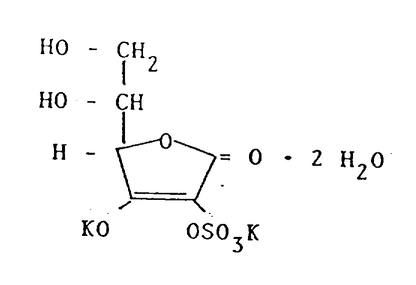 **characterized** in that a trimethylamine-sulphur trioxide complex is added in a slight molar excess of not more than 8%, preferably only 6%, to a concentrated solution of potassium ascorbate in water, the concentration of potassium ascorbate in water being from 500 to 750 g/l, whereafter the pH of this reaction mixture is adjusted to a value between 9.5 and 10.5 by successive additions of potassium hydroxide and maintained at this value for the duration of the reaction.

2. Method according to claim 1, **characterized** in that the pH is adjusted to a value of 10 and maintained for the duration of the reaction.

3. Method according to claim 1 or 2, **characterized in** that the temperature of the reaction mixture is set at a value ranging from 57°C to 65°C and preferably raised linearly during the course of the reaction from the above-mentioned lower temperature to the above-mentioned upper one.

4. Method according to claims 1 through 3, **characterized** in that the entire amount of trimethylamine-sulphur trioxide complex is added to the aqueous potassium ascorbate solution simultaneously.

5. Method according to claims 1 through 4, **characterized** in that the concentrated solution of potassium ascorbate in water is prepared by mixing L-ascorbic acid with water and neutralizing the mixture with potassium hydroxide.

6. Method according to claim 5, **characterized** in that the temperature during the preparation of the concentrated potassium ascorbate solution is maintained below 25°C.

7. Method according to claims 1 through 6, **characterized** in that the dipotassium ascorbate-2-sulphate dihydrate formed during the reaction is isolated from the reaction mixture by adding to it of an alkanol, such as methaol or ethanol, in particular ethanol.

8. Method according to claims 1 through 7, **characterized** in that the trimethylamine escaping from the reaction mixture during the reaction is absorbed in a chlorinated organic solvent, in particular 1,2-dichloroethane, chloroform, carbon tetrachloride, or methylene chloride.

9. Method according to claim 7, **characterized** in that the tertiary amine-sulphur trioxide complex absorbed or dissolved in the chlorinated organic solvent is recovered by adding chlorosulfonic acid to the solution and leading gaseous ammonium into the reaction mixture thus obtained for the purpose of recovering amine from the amine hydrochloride previously formed as a by-product.

10. Method according to claim 9, **characterized** in that ammonium is passed into the said reaction mixture at least until the pH of the latter is basic.

## Patentansprüche

1. Verfahren zum Herstellen von Dikalium-ascorbat-2-sulfat-Dihydrat der Formel 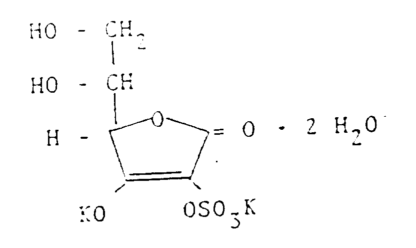 **dadurch gekennzeichnet**, daß ein Trimethylamin-Schwefeltrioxid-Komplex in leichtem molarem Überschuß von nicht mehr als 8%, vorzugsweise nur 6%, zu einer konzentrierten Lösung von Kaliumascorbat in Wasser zugegeben wird, wobei die Konzentration von Kaliumascorbat in Wasser 500 bis 750 g/l beträgt, wonach der pH dieses Reaktionsgemisches durch aufeinanderfolgende Zugaben von Kaliumhydroxid auf einen Wert zwischen 9,5 und 10,5 eingestellt und während der Dauer der Reaktion auf diesem Wert gehalten wird.

2. Verfahren nach Anspruch 1, **dadurch gekennzeichnet**, daß der pH auf den Wert 10 eingestellt und während der Dauer der Reaktion beibehalten wird.

3. Verfahren nach Anspruch 1 oder 2, **dadurch gekennzeichnet**, daß die Temperatur des Reaktionsgemisches auf einen Wert im Bereich von 57°C bis 65°C eingestellt und vorzugsweise im Laufe der Reaktion von der oben erwähnten niedrigeren Temperatur auf die oben erwähnte höhere Temperatur linear angehoben wird.

4. Verfahren nach den Ansprüchen 1 bis 3, **dadurch gekennzeichnet**, daß die gesamte Menge des Trimethylamin-Schwefeltrioxid-Komplexes der wäßrigen Kaliumascorbatlösung gleichzeitig zugegeben wird.

5. Verfahren nach den Ansprüchen 1 bis 4, **dadurch gekennzeichnet**, daß die konzentrierte Lösung von Kaliumascorbat in Wasser durch Vermischen von L-Ascorbinsäure mit Wasser und Neutralisieren des Gemisches mit Kaliumhydroxid hergestellt wird.

6. Verfahren nach Anspruch 5, **dadurch gekennzeichnet**, daß die Temperatur während der Herstellung der konzentrierten Kaliumascorbatlösung unter 25°C gehalten wird.

7. Verfahren nach den Ansprüchen 1 bis 6, **dadurch gekennzeichnet**, daß das während der Reaktion gebildete Dikaliumascorbat-2-sulfat-Dihydrat durch Zugabe eines Alkanols wie Methanol oder Ethanol, insbesondere Ethanol, zu dem Reaktionsgemisch aus dem Reaktionsgemisch isoliert wird.

8. Verfahren nach den Ansprüchen 1 bis 7, **dadurch gekennzeichnet**, daß das Trimethylamin, welches während der Reaktion aus dem Reaktionsgemisch entweicht, in einem chlorierten organischen Lösungsmittel, insbesondere 1,2-Dichlorethan, Chloroform, Tetrachlorkohlenstoff oder Methylenchlorid absorbiert wird.

9. Verfahren nach Anspruch 7, **dadurch gekennzeichnet**, daß der in dem chlorierten organischen Lösungsmittel absorbierte oder gelöste Komplex aus tertiärem Amin und Schwefeltrioxid durch Zugabe von Chlorsulfonsäure zu der Lösung und Einleiten von gasförmigem Ammoniak in das so erhaltene Reaktionsgemisch, um das Amin aus dem zuvor als Nebenprodukt gebildeten Aminhydrochlorid zu gewinnen, erhalten wird.

10. Verfahren nach Anspruch 9, **dadurch gekennzeichnet**, daß das Ammoniak in das Reaktionsgemisch mindestens so lange eingeleitet wird, bis der pH von letzterem basisch ist.

## Revendications

1. Procédé de production d'ascorbate-2-sulfate dipotassique dihydraté de formule : 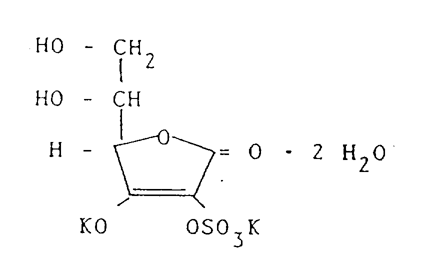 caractérisé en ce qu'on ajoute un complexe triméthylamine-trioxyde de soufre en un petit excès molaire inférieur ou égal à 8%, de préférence d'uniquement 6%, à une solution concentrée d'ascorbate de potassium dans l'eau, la concentration en ascorbate de potassium dans l'eau étant comprise entre 500 et 750 g/l, après quoi on ajuste le pH de ce mélange réactionnel à une valeur comprise entre 9,5 et 10,5 par ajouts successifs d'hydroxyde de potassium et on le maintient à cette valeur pendant la durée de la réaction.

2. Procédé selon la revendication 1, caractérisé en ce qu'on ajuste le pH à une valeur de 10 et en ce qu'on le maintient pendant la durée de la réaction.

3. Procédé selon la revendication 1 ou 2, caractérisé en ce que la température du mélange réactionnel est établie à une valeur comprise entre 57°C et 65°C et de préférence élevée linéairement au cours de la réaction de la température inférieure mentionnée ci-dessus jusqu'à la température supérieure mentionnée ci-dessus.

4. Procédé selon les revendications 1 à 3, caractérisé en ce qu'on ajoute la quantité totale du complexe triméthylamine-trioxyde de soufre à la solution aqueuse d'ascorbate de potassium simultanément.

5. Procédé selon les revendications 1 à 4, caractérisé en ce qu'on prépare la solution concentrée d'ascorbate de potassium dans l'eau en mélangeant de l'acide L-ascorbique avec de l'eau et en neutralisant le mélange avec de l'hydroxyde de potassium.

6. Procédé selon la revendication 5, caractérisé en ce que la température lors de la préparation de la solution concentrée d'ascorbate de potassium est maintenue à moins de 25°C.

7. Procédé selon les revendications 1 à 6, caractérisé en ce qu'on isole l'ascorbate-2-sulfate dipotassique dihydraté, formé lors de la réaction, à partir du mélange réactionnel en lui ajoutant un alcanol, tel que méthanol ou éthanol, en particulier l'éthanol.

8. Procédé selon les revendications 1 à 7, caractérisé en ce que la triméthylamine s'échappant du mélange réactionnel lors de la réaction est absorbée dans un solvant organique chloré, en particulier le 1,2-dichloroéthane, le chloroforme, le tétrachlorure de carbone ou le chlorure de méthylène.

9. Procédé selon la revendication 7, caractérisé en ce qu'on récupère le complexe amine tertiaire-trioxyde de soufre absorbé ou dissous dans le solvant organique chloré en ajoutant de l'acide chlorosulfonique à la solution et en apportant de l'ammonium gazeux dans le mélange réactionnel ainsi obtenu afin de récupérer l'amine du chlorhydrate d'amine formé au préalable sous la forme d'un sous-produit.

10. Procédé selon la revendication 9, caractérisé en ce qu'on fait passer l'ammonium dans ledit mélange réactionnel au moins jusqu'à ce que le pH du dernier soit basique.
